(19) Europäisches Patentamt
European Patent Office
Office européen des brevets

(11) Veröffentlichungsnummer: **0 141 995**
**A2**

(12) **EUROPÄISCHE PATENTANMELDUNG**

(21) Anmeldenummer: **84111705.4**

(22) Anmeldetag: **01.10.84**

(51) Int. Cl.⁴: **C 07 D 405/06**, A 01 N 43/50, A 01 N 43/653

(30) Priorität: **11.10.83 DE 3336859**

(43) Veröffentlichungstag der Anmeldung: **22.05.85 Patentblatt 85/21**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Elbe, Hans-Ludwig, Dr., Dasnöckel 59, D-5600 Wuppertal 11 (DE)**
Erfinder: **Jautelat, Manfred, Dr., Müllersbaum 28, D-5093 Burscheid (DE)**
Erfinder: **Büchel, Karl Heinz, Prof. Dr., Dabringhausener Strasse 42, D-5093 Burscheid (DE)**
Erfinder: **Brandes, Wilhelm, Dr., Eichendorffstrasse 3, D-5653 Leichlingen 1 (DE)**
Erfinder: **Hänssler, Gerd, Dr., Heymannstrasse 40, D-5090 Leverkusen 1 (DE)**
Erfinder: **Reinecke, Paul, Dr., Lessingstrasse 11, D-5090 Leverkusen 3 (DE)**

(54) **Substituierte Azolyl-tetrahydro-furan-2-yliden-methan-Derivate.**

(57) Substituierte Azolyltetrahydrofuran-2-ylidenmethan-Derivaten der allgemeinen Formel (I):

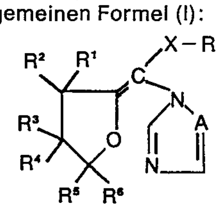

(I)

in welcher

A für ein Stickstoffatom oder die CH-Gruppe steht;

X für Sauerstoff, Schwefel, die SO- oder $SO_2$-Gruppe steht;

R für gegebenenfalls substituiertes Aryl steht;

$R^1$ bis $R^6$ gleich oder verschieden sind und für Wasserstoff, Alkyl, Halogenalkyl oder Halogen stehen, wobei maximal bis zu 3 Substituenten für Halogen bzw. Halogenalkyl stehen, mit der Massgabe, dass $R^1$ und $R^2$ nicht gleichzeitig für Methyl stehen, wenn X für Sauerstoff steht;

$R^1$ und $R^2$ gemeinsam mit dem Kohlenstoffatom, an das sie gebunden sind, für gegebenenfalls substituiertes Cycloalkyl stehen und

$R^2$ und $R^3$ gemeinsam mit den Kohlenstoffatomen, an die sie gebunden sind, für gegebenenfalls substituiertes Cycloalkyl stehen,

und deren Säureadditions-Salze und Metallsalz-Komplexe sowie ihre Verwendung als Fungizide.

Die neuen substituierten Azolyltetrahydrofuran-2-ylidenmethan-Derivate können z.B. hergestellt werden, indem man geeignete Halogen(thio)etherketone mit Imidazol oder Triazol umsetzt und gegebenenfalls die erhaltenen Verbindungen oxidiert.

EP 0 141 995 A2

BAYER AKTIENGESELLSCHAFT
Konzernverwaltung RP
Patentabteilung

5090 Leverkusen, Bayerwerk

Slr/Bas/by-c
Ia

Substituierte Azolyl-tetrahydro-furan-2-yliden-methan-Derivate

Die vorliegende Erfindung betrifft neue substituierte Azolyl-tetrahydrofuran-2-yliden-methan-Derivate, ein Verfahren zu ihrer Herstellung sowie ihre Verwendung als Fungizide.

Es ist bereits bekannt geworden, daß bestimmte Azolylalkenole, wie z.B. im Phenoxyteil substituierte 1-(Imidazol-1-yl)- bzw. 1-(1,2,4-Triazol-1-yl)-2-phenoxy-4,4-dimethyl-1-penten-3-ole, gute fungizide Eigenschaften aufweisen (vgl. DE-OS 29 28 967). Außerdem ist bereits bekannt, daß Disulfide, wie z.B. Zinkethylen-1,2-bisdithiocarbamidat, gute Mittel zur Bekämpfung von pilzlichen Pflanzenkrankheiten sind (vgl. R. Wegler, "Chemie der Pflanzenschutz- und Schädlingsbekämpfungsmittel", Band 2, S. 59 ff, Springer-Verlag 1970).

Die Wirkung dieser Verbindungen ist jedoch in bestimmten Indikationsbereichen, insbesondere bei niedrigen Aufwandmengen und -konzentrationen, nicht immer voll befriedigend.

Es wurden neue substituierte Azolyl-tetrahydrofuran-2-yliden-methan-Derivate der allgemeinen Formel (I)

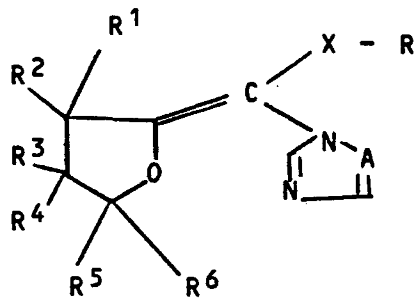

(I)

in welcher

A für ein Stickstoffatom oder die CH-Gruppe steht;

X für Sauerstoff, Schwefel, die SO- oder $SO_2$-Gruppe steht;

R für gegebenenfalls substituiertes Aryl steht;

$R^1$ bis $R^6$ gleich oder verschieden sind und für Wasserstoff, Alkyl, Halogenalkyl oder Halogen stehen, wobei maximal bis zu 3 Substituenten für Halogen bzw. Halogenalkyl stehen, mit der Maßgabe, daß $R^1$ und $R^2$ nicht gleichzeitig für Methyl stehen, wenn X für Sauerstoff steht;

$R^1$ und $R^2$ gemeinsam mit dem Kohlenstoffatom, an das sie gebunden sind, für gegebenenfalls substituiertes Cycloalkyl stehen und

$R^2$ und $R^3$ gemeinsam mit den Kohlenstoffatomen, an die sie gebunden sind, für gegebenenfalls substituiertes Cycloalkyl stehen,

und deren Säureadditions-Salze und Metallsalz-Komplexe gefunden.

Die Verbindungen der Formel (I) können in zwei geometrischen Isomerenformen vorliegen, je nach Anordnung der Gruppen, die an die Doppelbindung gebunden sind; vorzugsweise fallen sie in einem wechselnden Isomerenverhältnis an. Die vorliegende Erfindung betrifft sowohl die einzelnen Isomeren als auch die Isomerengemische.

Weiterhin wurde gefunden, daß man die substituierten Azolyl-tetrahydrofuran-2-yliden-methan-Derivate der Formel (I) erhält, wenn man

Halogen-(thio)ether-ketone der Formel (II)

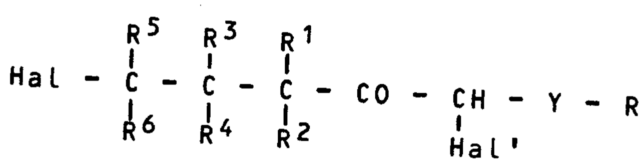

(II)

in welcher

| | |
|---|---|
| R und $R^1$ bis $R^6$ | die oben angegebene Bedeutung haben, |
| Y | für Sauerstoff oder Schwefel steht und |
| Hal und Hal' | für Halogen, vorzugsweise Chlor oder Brom stehen, |

mit Imidazol oder 1,2,4-Triazol in Gegenwart eines Verdünnungsmittels und in Gegenwart eines Säurebindemittels umsetzt; und gegebenenfalls die so erhaltenen substituierten Azolyl-tetrahydrofuran-2-yliden-methan-Derivate der Formel (Ia)

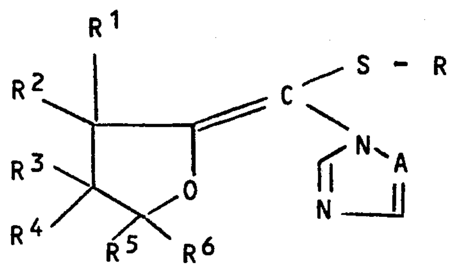

(Ia)

in welcher

A,R und $R^1$ bis $R^6$ die oben angegebene Bedeutung haben,

nach bekannten Methoden in üblicher Weise oxidiert.

An die so erhaltenen Verbindungen der Formel (I) kann gegebenenfalls anschließend eine Säure oder ein Metallsalz addiert werden.

Außerdem wurde gefunden, daß die neuen substituierten Azolyl-tetrahydrofuran-2-yliden-methan-Derivate der Formel (I) starke fungizide Eigenschaften aufweisen.

Überraschenderweise zeigen die erfindungsgemäßen Verbindungen der Formel (I) bessere fungizide Wirkungen als die aus dem Stand der Technik bekannten, im Phenoxyteil substituierten 1-(Imidazol-1-yl)- bzw.

1-(1,2,4-Triazol-1-yl)-2-phenoxy-4,4-dimethyl-1-penten-3-ole und als das ebenfalls bekannte Zinkethylen-1,2-bisdithiocarbamidat. Die erfindungsgemäßen Wirkstoffe stellen somit eine Bereicherung der Technik dar.

Die erfindungsgemäßen substituierten Azolyl-tetrahydrofuran-2-yliden-methan-Derivate sind durch die Formel (I) allgemein definiert. In dieser Formel stehen vorzugsweise

A für ein Stickstoffatom oder die CH-Gruppe;

X für Sauerstoff, Schwefel, die SO- oder $SO_2$-Gruppe;

R für gegebenenfalls einfach bis dreifach, gleich oder verschieden substituiertes Phenyl, wobei als Substituenten genannt seien: Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, wie Fluor- und Chloratomen, Alkoxycarbonyl mit 1 bis 4 Kohlenstoffatomen im Alkylteil, Alkoximinoalkyl mit 1 bis 4 Kohlenstoffatomen im Alkoxyteil und 1 oder 2 Kohlenstoffatomen im Alkylteil, Nitro, Cyano oder gegebenenfalls durch Halogen und/oder Alkyl mit 1 oder 2 Kohlenstoffatomen substituiertes Phenyl oder Phenoxy;

$R^1$ bis $R^6$, die gleich oder verschieden sein können, für Wasserstoff, geradkettiges oder verzweigtes Alkyl mit 1 bis 4 Kohlenstoffatomen oder für Halogenalkyl mit 1 oder 2 Kohlenstoffatomen und 1 bis 5 gleichen oder verschiedenen Halogenatomen, wie Fluor- und Chloratomen, wobei maximal bis zu 3 Substituenten für Halogenalkyl stehen, mit der Maßgabe, daß $R^1$ und $R^2$ nicht gleichzeitig für Methyl stehen, wenn X für Sauerstoff steht;

$R^3$ bis $R^6$, die gleich oder verschieden sein können, außerdem für Halogen, wobei maximal bis zu 3 Substituenten diese Bedeutung haben;

$R^1$ und $R^2$ gemeinsam mit dem Kohlenstoffatom, an das sie gebunden sind, für gegebenenfalls durch Alkyl mit 1 oder 2 Kohlenstoffatomen substituiertes Cycloalkyl mit 4 bis 7 Kohlenstoffatomen; und

$R^2$ und $R^3$ gemeinsam mit den Kohlenstoffatomen, an die sie gebunden sind, für gegebenenfalls durch Alkyl mit 1 oder 2 Kohlenstoffatomen substituiertes Cycloalkyl mit 4 bis 7 Kohlenstoffatomen.

Besonders bevorzugt sind diejenigen Verbindungen der Formel (I), in denen

A für ein Stickstoffatom oder die CH-Gruppe steht;

X für Sauerstoff, Schwefel, die SO- oder $SO_2$-Gruppe steht;

R für gegebenenfalls einfach bis dreifach, gleich oder verschieden substituiertes Phenyl steht, wobei als Substituenten genannt seien: Fluor, Chlor, Brom, Methyl, Ethyl, tert.-Butyl, Methoxy, Methylthio, Trifluormethyl, Trifluormethoxy, Trifluormethylthio, Methoxycarbonyl, Ethoxycarbonyl, Methoximinomethyl, 1-Methoximinoethyl, Nitro, Cyano, gegebenenfalls durch Chlor und/oder Methyl substituiertes Phenyl oder Phenoxy;

$R^1$ bis $R^6$ die gleich oder verschieden sein können, für Wasserstoff, Methyl, Ethyl oder Halogenmethyl mit 1 bis 3 gleichen oder verschiedenen Halogenatomen, wie Fluor- und Chloratomen, stehen, wobei maximal drei Substituenten für Halogenmethyl stehen, mit der Maßgabe, daß $R^1$ und $R^2$ nicht gleichzeitig für Methyl stehen, wenn X für Sauerstoff steht;

$R^3$ bis $R^6$, die gleich oder verschieden sein können, außerdem für Fluor, Chlor oder Brom stehen, wobei maximal bis zu drei Substituenten diese Bedeutung haben;

$R^1$ und $R^2$ gemeinsam mit dem Kohlenstoffatom, an das sie gebunden sind, für Cyclopentyl oder Cyclohexyl stehen; und

$R^2$ und $R^3$ gemeinsam mit den Kohlenstoffatomen, an die sie gebunden sind, für Cyclopentyl oder Cyclohexyl stehen.

Im einzelnen seien außer den bei den Herstellungsbeispielen genannten Verbindungen die folgenden Verbindungen der allgemeinen Formel (I) genannt:

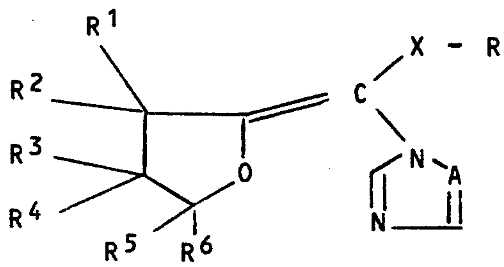

(I)

(wobei A sowohl für ein Stickstoffatom als auch die CH-Gruppe steht).

| $R^1$ | $R^2$ | $R^3$ | $R^4$ | $R^5$ | $R^6$ | X | R |
|---|---|---|---|---|---|---|---|
| $C_2H_5$ | $CH_3$ | H | H | H | H | O | 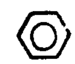 |
| $C_2H_5$ | $CH_3$ | H | H | H | H | S | 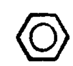 |
| $-(CH_2)_5-$ | | H | H | H | H | O | 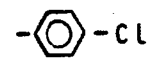 |
| $C_2H_5$ | $CH_3$ | H | H | H | H | O | 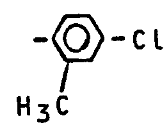 |
| $C_2H_5$ | $CH_3$ | H | H | H | H | O | 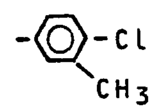 |
| $C_2H_5$ | $CH_3$ | H | H | H | H | O | 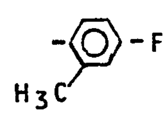 |
| $C_2H_5$ | $CH_3$ | H | H | H | H | O | 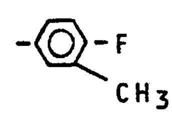 |
| $C_2H_5$ | $CH_3$ | H | H | H | H | O | 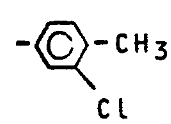 |

| $R^1$ | $R^2$ | $R^3$ | $R^4$ | $R^5$ | $R^6$ | X | R |
|---|---|---|---|---|---|---|---|
| $C_2H_5$ | $CH_3$ | H | H | H | H | O | 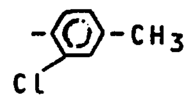 |
| $C_2H_5$ | $CH_3$ | H | H | H | H | O | 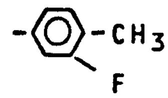 |
| $C_2H_5$ | $CH_3$ | H | H | H | H | O | 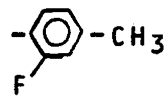 |
| $C_2H_5$ | $CH_3$ | H | H | H | H | O | 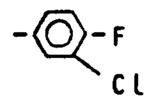 |
| $C_2H_5$ | $CH_3$ | H | H | H | H | O | 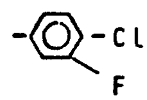 |
| $C_2H_5$ | $CH_3$ | H | H | H | H | O | 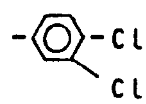 |
| $C_2H_5$ | $CH_3$ | H | H | H | H | O | 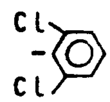 |
| $C_2H_5$ | $CH_3$ | H | H | H | H | O | 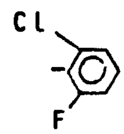 |
| $C_2H_5$ | $CH_3$ | H | H | H | H | O | 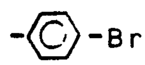 |
| $C_2H_5$ | $CH_3$ | H | H | H | H | O | 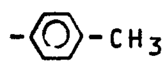 |
| $C_2H_5$ | $CH_3$ | H | H | H | H | O | 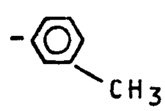 |
| $C_2H_5$ | $CH_3$ | H | H | H | H | O | 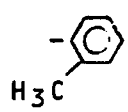 |
| $C_2H_5$ | $CH_3$ | H | H | H | H | O | 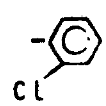 |
| $C_2H_5$ | $CH_3$ | H | H | H | H | O | 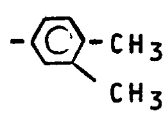 |

| $R^1$ | $R^2$ | $R^3$ | $R^4$ | $R^5$ | $R^6$ | X | R |
|---|---|---|---|---|---|---|---|
| $C_2H_5$ | $CH_3$ | H | H | H | H | O | 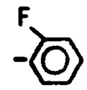 |
| $C_2H_5$ | $CH_3$ | H | H | H | H | O | 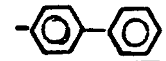 |
| $C_2H_5$ | $CH_3$ | H | H | H | H | O | 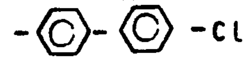 |
| $C_2H_5$ | $CH_3$ | H | H | H | H | O | 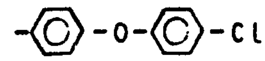 |
| $C_2H_5$ | $CH_3$ | H | H | H | H | O | 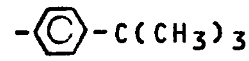 |
| $C_2H_5$ | $CH_3$ | H | H | H | H | O | 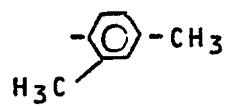 |
| $C_2H_5$ | $CH_3$ | H | H | H | H | O | 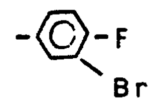 |
| $C_2H_5$ | $CH_3$ | H | H | H | H | O | 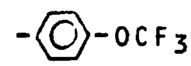 |
| $C_2H_5$ | $CH_3$ | H | H | H | H | O | 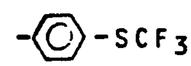 |
| $C_2H_5$ | $CH_3$ | H | H | H | H | O | 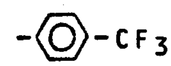 |
| $C_2H_5$ | $CH_3$ | H | H | H | H | O | 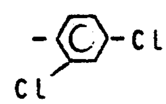 |
| $C_2H_5$ | $CH_3$ | H | H | H | H | O | 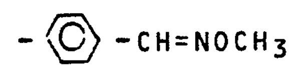 |
| $C_2H_5$ | $CH_3$ | H | H | H | H | O | 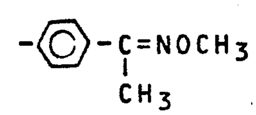 |
| $C_2H_5$ | $CH_3$ | H | H | H | H | S | 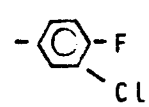 |
| $C_2H_5$ | $CH_3$ | H | H | H | H | S | 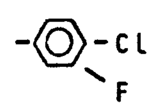 |
| $C_2H_5$ | $CH_3$ | H | H | H | H | S | 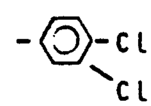 |

| $R^1$ | $R^2$ | $R^3$ | $R^4$ | $R^5$ | $R^6$ | X | R |
|---|---|---|---|---|---|---|---|
| $C_2H_5$ | $CH_3$ | H | H | H | H | S | 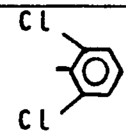 |
| $C_2H_5$ | $CH_3$ | H | H | H | H | S | 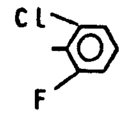 |
| $C_2H_5$ | $CH_3$ | H | H | H | H | S | 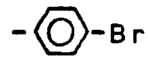 |
| $C_2H_5$ | $CH_3$ | H | H | H | H | S | 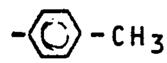 |
| $C_2H_5$ | $CH_3$ | H | H | H | H | S | 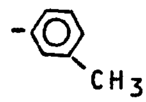 |
| $C_2H_5$ | $CH_3$ | H | H | H | H | S | 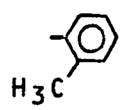 |
| $C_2H_5$ | $CH_3$ | H | H | H | H | S | 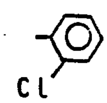 |
| $C_2H_5$ | $CH_3$ | H | H | H | H | S | 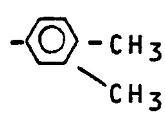 |
| $C_2H_5$ | $CH_3$ | H | H | H | H | S | 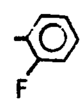 |
| $C_2H_5$ | $CH_3$ | H | H | H | H | S | 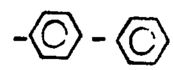 |
| $C_2H_5$ | $CH_3$ | H | H | H | H | S | 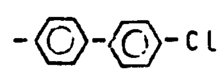 |
| $C_2H_5$ | $CH_3$ | H | H | H | H | S | 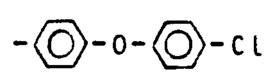 |
| $C_2H_5$ | $CH_3$ | H | H | H | H | S | 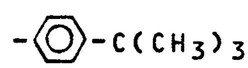 |
| $C_2H_5$ | $CH_3$ | H | H | H | H | S | 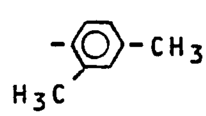 |

| $R^1$ | $R^2$ | $R^3$ | $R^4$ | $R^5$ | $R^6$ | X | R |
|---|---|---|---|---|---|---|---|
| $C_2H_5$ | $CH_3$ | H | H | H | H | S | 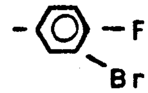 |
| $C_2H_5$ | $CH_3$ | H | H | H | H | S | 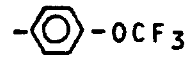 |
| $C_2H_5$ | $CH_3$ | H | H | H | H | S | 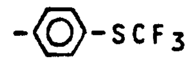 |
| $C_2H_5$ | $CH_3$ | H | H | H | H | S | 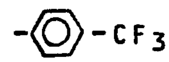 |
| $C_2H_5$ | $CH_3$ | H | H | H | H | S | 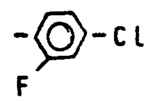 |
| $C_2H_5$ | $CH_3$ | H | H | H | H | S | 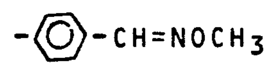 |
| $C_2H_5$ | $CH_3$ | H | H | H | H | S | 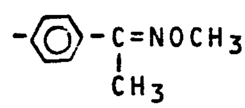 |
| $C_2H_5$ | $CH_3$ | H | H | H | H | S | 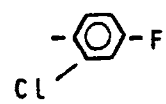 |
| $C_2H_5$ | $CH_3$ | H | H | H | H | S | 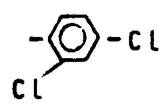 |
| $C_2H_5$ | $C_2H_5$ | H | H | H | H | O | 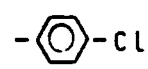 |
| $C_2H_5$ | $C_2H_5$ | H | H | H | H | O | 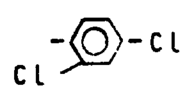 |
| $C_2H_5$ | $C_2H_5$ | H | H | H | H | O | 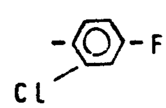 |
| $C_2H_5$ | $C_2H_5$ | H | H | H | H | O | 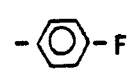 |
| $C_2H_5$ | $C_2H_5$ | H | H | H | H | S | 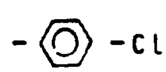 |

| $R^1$ | $R^2$ | $R^3$ | $R^4$ | $R^5$ | $R^6$ | X | R |
|---|---|---|---|---|---|---|---|
| $C_2H_5$ | $C_2H_5$ | H | H | H | H | S | 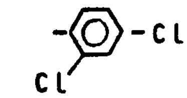 |
| $C_2H_5$ | $C_2H_5$ | H | H | H | H | S | 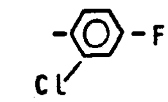 |
| $C_2H_5$ | $C_2H_5$ | H | H | H | H | S | 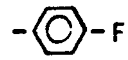 |
| $C_2H_5$ | $CH_3$ | H | H | H | H | S | 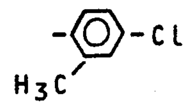 |
| $C_2H_5$ | $CH_3$ | H | H | H | H | S | 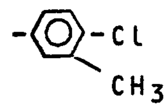 |
| $C_2H_5$ | $CH_3$ | H | H | H | H | S | 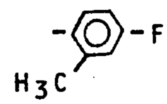 |
| $C_2H_5$ | $CH_3$ | H | H | H | H | S | 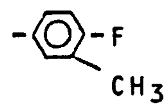 |
| $C_2H_5$ | $CH_3$ | H | H | H | H | S | 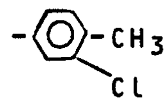 |
| $C_2H_5$ | $CH_3$ | H | H | H | H | S | 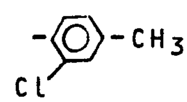 |
| $C_2H_5$ | $CH_3$ | H | H | H | H | S | 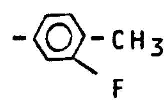 |
| $C_2H_5$ | $CH_3$ | H | H | H | H | S | 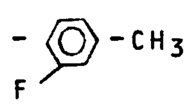 |

Bevorzugte erfindungsgemäße Verbindungen sind auch Additionsprodukte aus Säuren und denjenigen substituierten Azolyltetrahydrofuran-2-yliden-methan-Derivaten der Formel (I), in denen die Substituenten A, X, R und $R^1$ bis $R^6$ die Bedeutungen haben, die bereits vorzugsweise für diese Substituenten genannt wurden.

Zu den Säuren, die addiert werden können, gehören vorzugsweise Halogenwasserstoffsäuren, wie z.B. Chlorwasserstoffsäure und Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen substituierten Azolyl-tetrahydrofuran-2-yliden-methan-Derivaten der Formel (I), in denen die Substituenten A, X, R und $R^1$ bis $R^6$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure.

Verwendet man beispielsweise 1-Brom-1-(4-chlorphenylthio)-5-chlor-3,3-dimethyl-2-pentanon und Imidazol als Ausgangsstoffe, so kann der Reaktionsablauf durch das folgende Formelschema wiedergegeben werden:

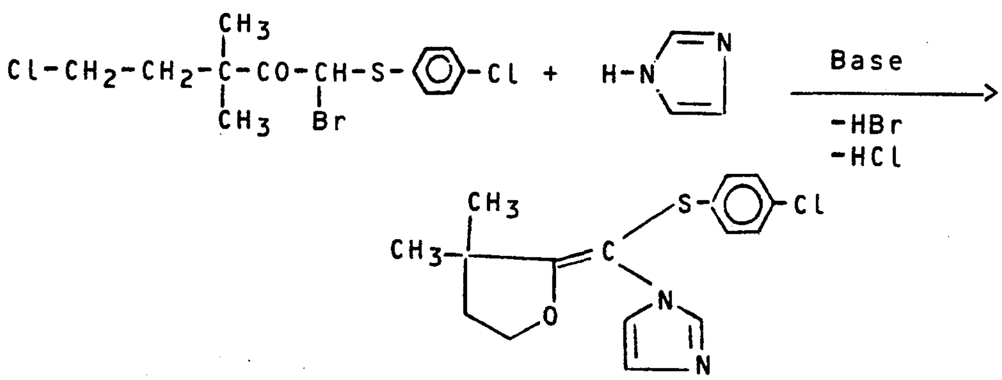


Verwendet man beispielsweise (4-Chlorphenylthio)-(imidazol-1-yl)-3,3-dimethyltetrahydrofuran-2-ylidenmethan und Wasserstoffperoxid in Eisessig als Ausgangsstoffe, so kann der Reaktionsablauf der erfindungsgemäßen Oxidation durch das folgende Formelschema wiedergegeben werden:

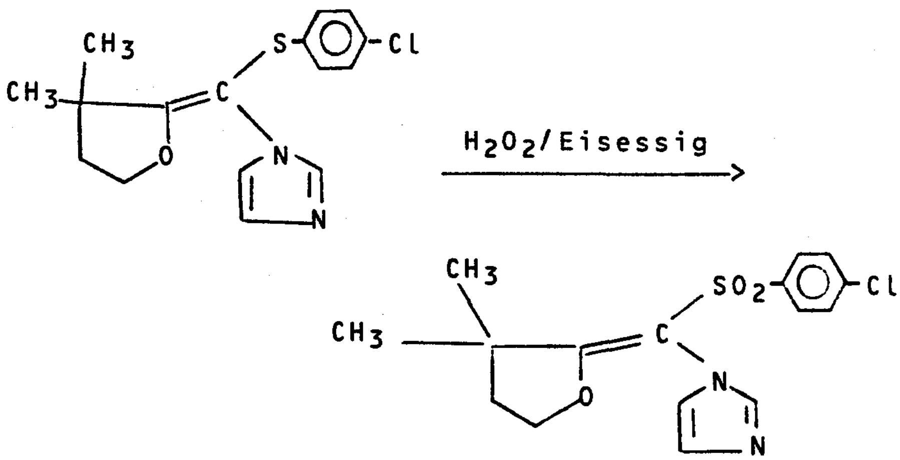


Die bei der Durchführung des erfindungsgemäßen Verfahrens als Ausgangsstoffe zu verwendenden Halogen-(thio)ether-ketone sind durch die Formel (II) allgemein definiert. In dieser Formel stehen R und $R^1$ bis $R^6$ vorzugsweise für die Reste, die bereits im Zusammenhang mit der Beschreibung der erfindungsgemäßen Stoffe der Formel (I) vorzugsweise für diese Substituenten genannt wurden.

Die Halogen-(thio)ether-ketone der Formel (II) sind noch nicht bekannt; sie können jedoch nach bekannten Verfahren hergestellt werden, indem man Halogenketone der Formel (III)

$$Hal - \underset{R^6}{\overset{R^5}{\underset{|}{\overset{|}{C}}}} - \underset{R^4}{\overset{R^3}{\underset{|}{\overset{|}{C}}}} - \underset{R^2}{\overset{R^1}{\underset{|}{\overset{|}{C}}}} - CO - CH_2 - Hal'' \qquad (III)$$

in welcher

$R^1$ bis $R^6$ und Hal die oben angegebene Bedeutung haben und

Hal" für Halogen, vorzugsweise Chlor oder Brom, steht

mit bekannten (Thio)Phenolen der Formel (IV)

$$H - Y - R \qquad (IV)$$

in welcher

R und Y die oben angegebene Bedeutung haben,

in üblicher Weise umsetzt und in den entstehenden (Thio)Ether-Ketonen der Formel (V)

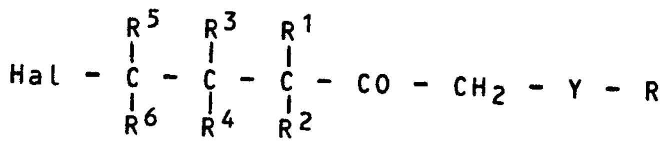

(V)

in welcher

Hal, R, $R^1$ bis $R^6$ und Y die oben angegebene Bedeutung haben,

das verbleibende aktive Wasserstoffatom in üblicher Weise gegen Halogen austauscht (vgl. auch die Herstellungsbeispiele). Die Halogen-(thio)ether-ketone der Formel (II) können gegebenenfalls ohne Isolierung direkt weiter umgesetzt werden.

Die Halogenketone der Formel (III) sind teilweise bekannt (vgl. DE-OS 32 04 788) bzw. können sie nach dem dort angegebenen Verfahren erhalten werden, indem man 2-Chlormethylen-tetrahydrofurane der Formel (VI)

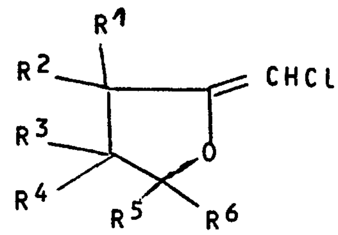

(VI)

in welcher

$R^1$ bis $R^6$ die oben angegebene Bedeutung haben,

mit aciden Verbindungen der Formel (VII)

H - Hal (VII)

in welcher

Hal die oben angegebene Bedeutung hat,

gegebenenfalls in Gegenwart eines inerten organischen Lösungsmittels, wie beispielsweise Toluol oder Methylenchlorid, bei Temperaturen zwischen 20 und 150°C umsetzt.

Die 2-Chlormethylen-tetrahydrofurane der Formel (VI) sind ebenfalls noch nicht bekannt; sie können jedoch in bekannter Art und Weise erhalten werden (vgl. DE-OS 32 04 692), indem man z.B. 1,1,5-Trichlor-penten-Derivate der Formel (VIII)

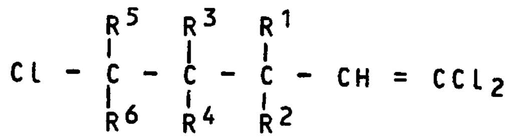 (VIII)

in welcher

$R^1$ bis $R^6$ die oben angegebene Bedeutung haben,

oder 1,1,1,5-Tetrachlor-pentan-Derivate der Formel (IX)

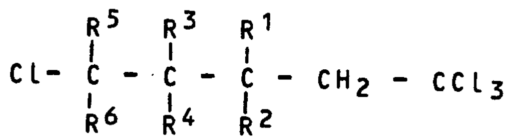 (IX)

in welcher

$R^1$ bis $R^6$ die oben angegebene Bedeutung haben,

mit Carboxylaten, wie beispielsweise wasserfreiem Natriumacetat, und mit Basen, wie beispielsweise Natriummethylat, in Gegenwart eines inerten organischen Lösungsmittels, wie beispielsweise Dimethylformamid, unter Rückflußtemperatur umsetzt.

Bestimmte Halogenketone der Formel (III) können auch erhalten werden, indem man entsprechende (ungesättigte) Ketone in üblicher Weise mit Halogenierungsmitteln, wie beispielsweise Brom, Bromwasserstoff oder Sulfurylchlorid umsetzt (vgl. hierzu auch die Herstellungsbeispiele).

Für die erfindungsgemäße Umsetzung kommen als Verdünnungsmittel inerte organische Lösungsmittel infrage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; aromatische Kohlenwasserstoffe, wie Toluol, Benzol oder Chlorbenzol; Formamide, wie insbesondere Dimethylformamid; und halogenierte Kohlenwasserstoffe.

Die erfindungsgemäße Umsetzung wird in Gegenwart eines Säurebindemittels vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden Überschuß an Azol.

Die Reaktionstemperaturen können bei der erfindungsgemäßen Umsetzung in einem größeren Bereich variiert werden. Im allgemeinen arbeitet man zwischen etwa 20 bis etwa 150°C, vorzugsweise bei 60 bis 120°C.

Bei der Durchführung der erfindungsgemäßen Umsetzung setzt man auf 1 Mol der Verbindung der Formel (II)

vorzugsweise 1 bis 4 Mol Azol und 1 bis 4 Mol Säurebinder ein. Zur Isolierung der Verbindungen der Formel (I) wird das Lösungsmittel abdestilliert und der Rückstand in üblicher Art und Weise aufgearbeitet.

Die Reaktionstemperaturen können bei der Durchführung der erfindungsgemäßen Oxidation in einem größeren Bereich variiert werden. Im allgemeinen arbeitet man zwischen etwa -50 bis 100°C, vorzugsweise zwischen -30 und 80°C.

Bei der Durchführung der erfindungsgemäßen Oxidation setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen -30°C bis +30°C, entstehen vorzugsweise die erfindungsgemäßen Verbindungen der Formel (I) mit der -SO-Gruppierung. Bei Überschuß an Oxidationsmittel und höheren Temperaturen (10 bis 80°C) entstehen vorzugsweise die erfindungsgemäßen Verbindungen der Formel (I) mit der $-SO_2$-Gruppierung. Die Isolierung der Oxidationsprodukte erfolgt in üblicher Weise.
Die Säureadditionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren

isoliert und gegebenenfalls durch Waschen mit einem organischen Lösungsmittel gereinigt werden.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Verbindungen der Formel (I) für X = O oder S können auch erhalten werden, wenn man

a) Halogenketone der Formel (III) mit Imidazol oder 1,2,4-Triazol gemäß den Bedingungen des erfindungsgemäßen Verfahrens umsetzt, anschließend die so erhaltenen Azolyl-tetrahydrofuran-2-yliden-methane der Formel (X)

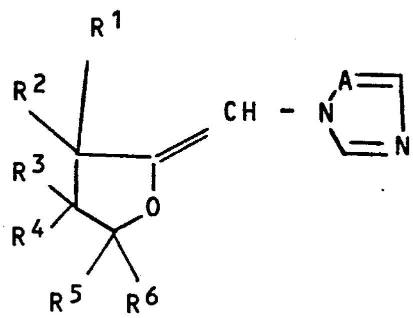

(X)

in welcher

A und $R^1$ bis $R^6$ die oben angegebene Bedeutung haben,

zunächst mit Halogen, insbesondere mit Brom, und danach mit (Thio)Phenolen der Formel (IV) jeweils in üblicher Weise umsetzt; oder

b) 2-Chlormethylen-tetrahydrofurane der Formel (VI) in üblicher Weise mit (Thio)Phenolen der Formel (IV) umsetzt, anschließend die so erhaltenen (Thio)Phenoxytetrahydrofuran-2-yliden-methane der Formel (XI)

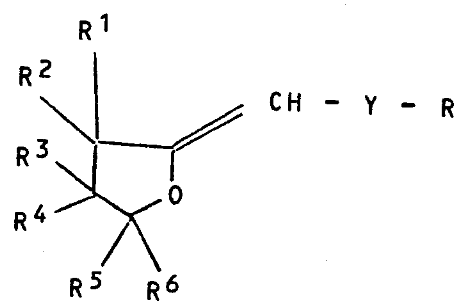

(XI)

in welcher

R, $R^1$ bis $R^6$ und Y die oben angegebene Bedeutung haben,

zunächst in üblicher Weise mit Halogen, insbesondere mit Brom, und danach gemäß den Bedingungen des erfindungsgemäßen Verfahrens mit Imidazol oder 1,2,4-Triazol umsetzt.

Die Azolyl-tetrahydrofuran-2-yliden-methane der Formel (X) und die (Thio)Phenoxytetrahydrofuran-2-yliden-methane der Formel (XI) sind neu; sie stellen allgemein interessante Zwischenprodukte dar.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Sphaerotheca-Arten, wie gegen den Erreger des echten Gurkenmehltaus (Sphaerotheca fuliginea); von Botrytis-Arten, wie gegen den Erreger des Grauschimmels (Botrytis cinerea); von Getreidekrankheiten, wie Erysiphe graminis, Roste, Cochliobolus sativus oder Pyrenophora teres; sowie von Reiskrankheiten, wie Pyricularia oryzae und Pellicularia sasakii, eingesetzt werden. Außerdem zeigen die erfindungsgemäßen Stoffe ein breites und gutes fungizides in-vitro-Wirkungsspektrum.
Hervorzuheben ist, daß die erfindungsgemäßen Stoffe nicht nur eine protektive Wirkung entfalten, sondern teilweise

auch systemisch sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe,

wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertigen Lösungen,

Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Herstellungsbeispiele

Beispiel 1

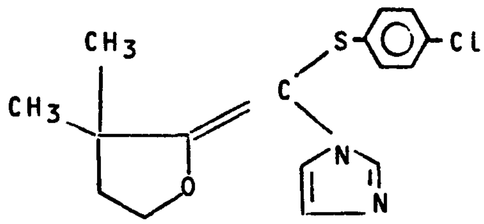

(I-1)

64,9 g (0,94 Mol) Imidazol und 129,7 g (0,94 Mol) Kaliumcarbonat werden in 1300 ml Toluol gelöst. Diese Mischung wird bei 80°C mit 173,9 g (0,47 Mol) 1-Brom-1-(4-chlorphenylthio)-5-chlor-3,3-dimethyl-2-pentanon in 360 ml Toluol versetzt. Man läßt das Reaktionsgemisch 15 Stunden bei 90°C nachrühren, kühlt ab und saugt vom anorganischen Rückstand ab. Das Filtrat wird mit Wasser gewaschen, über Natriumsulfat getrocknet und eingeengt. Der Rückstand wird aus Diethylether umkristallisiert. Man erhält 61,4 g (40,8 % der Theorie) (4-Chlorphenylthio)-(imidazol-1-yl)-(3,3-dimethyl-tetrahydrofuran-2-yliden)-methan vom Schmelzpunkt 134°C.

Herstellung des Ausgangsproduktes

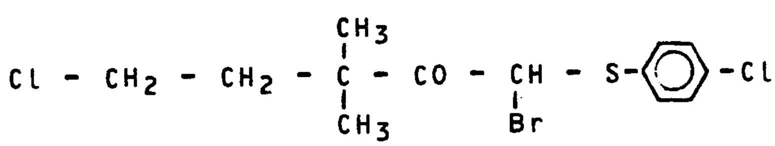

(II-1)

137 g (0,47 Mol) 1-(4-Chlorphenylthio)-5-chlor-3,3-dimethyl-2-pentanon werden in 1000 ml Chloroform gelöst. Man läßt bei Raumtemperatur 75,3 g (0,47 Mol) Brom so zutropfen, daß sich die Lösung immer entfärbt. Anschließend wird 1 Stunde bei Raumtemperatur nachgerührt und das Reaktionsgemisch durch Abdestillieren des Lösungsmittels eingeengt. Man erhält 172,7 g (99,3 % der Theorie) 1-Brom-1-(4-chlorphenylthio)-5-chlor-3,3-dimethyl-2-pentanon vom Brechungsindex $n_D^{20}$ = 1,5796.

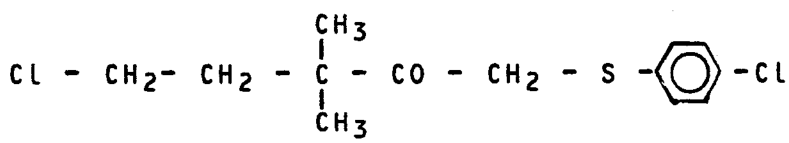

(V-1)

86,7 g (0,6 Mol) 4-Chlorthiophenol und 82,8 g (0,6 Mol) Kaliumcarbonat in 500 ml Toluol werden 2 Stunden unter Rückfluß am Wasserabscheider erhitzt. Bei 100°C versetzt man anschließend mit 91,5 g (0,5 Mol) 1,5-Dichlor-3,3-dimethyl-2-pentanon in 200 ml Toluol. Das Reaktionsgemisch wird 5 Stunden bei 100°C gerührt, danach läßt man abkühlen und verrührt mit 500 ml Wasser. Die organische Phase wird abgetrennt, mit verdünnter Natronlauge und mit Wasser gewaschen, über Natriumsulfat getrocknet und eingeengt. Man erhält 137,4 g (94,4 % der Theorie) 1-(4-Chlorphenylthio)-5-chlor-3,3-dimethyl-2-pentanon vom Brechungsindex $n_D^{20}$ = 1,5628.

$$Cl - CH_2 - CH_2 - \overset{CH_3}{\underset{CH_3}{\overset{|}{\underset{|}{C}}}} - CO - CH_2 - Cl \qquad (III-1)$$

In 476 g (3,25 Mol) 2-Chlormethylen-3,3-dimethyltetrahydrofuran wird unter Eiskühlung ein starker Chlorwasserstoffgasstrom aus einer Bombe eingeleitet. Das Gas wird vollständig absorbiert und die Innentemperatur steigt bis auf 30°C. Nach vollständiger Sättigung mit Chlorwasserstoff wird das Reaktionsgemisch 2 Stunden bei Raumtemperatur nachgerührt. Überschüssiger Chlorwasserstoff wird zunächst in die Wasserstrahlpumpe gezogen, dann wird bei gutem Vakuum destilliert. Man erhält 531 g (90 % der Theorie) 1,5-Dichlor-3,3-dimethyl-2-pentanon vom Siedepunkt 85-90°C/0,3 mbar.

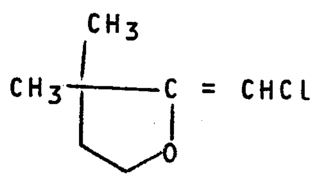

(VI-1)

806 g (4 Mol) 1,1,5-Trichlor-3,3-dimethyl-1-penten werden mit 360 g (4,4 Mol) wasserfreiem Natriumacetat in 1 l Dimethylformamid 6 Stunden unter Rückfluß erhitzt. Nach Abkühlung auf ca. 100°C tropft man 1,6 l (8 Mol) 30 %ige Natriummethylatlösung in Methanol ein und erhitzt weitere 4 Stunden unter Rückfluß. Die kalte Lösung wird in Wasser gegossen und mit Methylenchlorid mehrfach extrahiert.

Nach Trocknen der Lösung und Abdestillieren des Lösungsmittels bleiben 654 g Produkt zurück, das über eine Kolonne fraktioniert wird. Man erhält 522 g (89 % der Theorie) 2-Chlormethylen-3,3-dimethyltetrahydrofuran vom Siedepunkt 84-87°C/20 mbar.

Beispiel 2

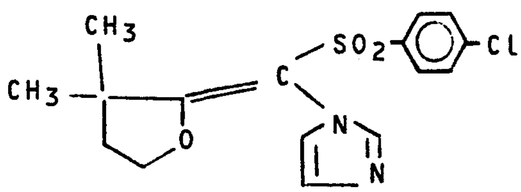

(I-2)

20 g (0,062 Mol) (4-Chlorphenylthio)-(imidazol-1-yl)-(3,3-dimethyl-tetrahydrofuran-2-yliden)-methan (Beispiel I-1) und 28 g (0,25 Mol) 30 %iges Wasserstoffperoxid in 200 ml Eisessig werden 20 Stunden bei 50°C gerührt. Anschließend wird das Reaktionsgemisch in 1,5 l Wasser gegeben und mit Methylenchlorid extrahiert. Die organische Phase wird über Natriumsulfat getrocknet und eingeengt. Man erhält 10,7 g (49 % der Theorie) (4-Chlorphenylsulfonyl)-(imidazol-1-yl)-(3,3-dimethyl-tetrahydrofuran-2-yliden)-methan vom Schmelzpunkt 192-194°C.

Beispiel 3

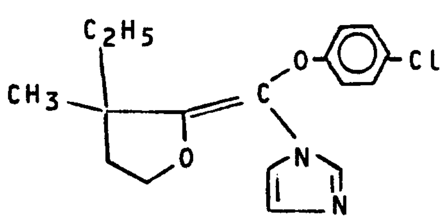

(I-3)

69,9 g (0,19 Mol) 1-Brom-1-(4-chlorphenoxy)-5-chlor-3-ethyl-3-methyl-2-pentanon, 26,2 g (0,38 Mol) Imidazol und 52,4 g (0,38 Mol) Kaliumcarbonat in 500 ml Toluol werden 10 Stunden auf 90°C erhitzt. Danach läßt man abkühlen und gibt das Reaktionsgemisch auf 500 ml Wasser. Die organische Phase wird abgetrennt, mit Wasser gewaschen, über Natriumsulfat getrocknet und eingeengt. Der Rückstand wird säulenchromatographisch (Essigester/Cyclohexan = 3:1) gereinigt. Man erhält 8,5 g (14 % der Theorie) (4-Chlorphenoxy)-(imidazol-1-yl)-(3-ethyl-3-methyl-tetrahydrofuran-2-yliden)-methan vom Brechungsindex $n_D^{20}$ = 1,5552.

Herstellung des Ausgangsproduktes

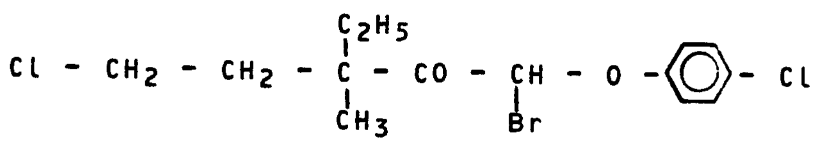 (II-2)

110 g (0,38 Mol) 1-(4-Chlorphenoxy)-5-chlor-3-ethyl-3-methyl-2-pentanon werden in 400 ml Chloroform gelöst. Man läßt bei Raumtemperatur 60,8 g (0,38 Mol) Brom so zutropfen, daß sich die Lösung immer entfärbt. Anschließend wird 30 Minuten bei Raumtemperatur nachgerührt und das Reaktionsgemisch durch Abdestillieren des Lösungsmittels eingeengt. Man erhält 138,5 g (99 % der Theorie) 1-Brom-1-(4-chlorphenoxy)-5-chlor-3-ethyl-3-methyl-2-pentanon vom Brechungsindex $n_D^{20}$= 1,5250.

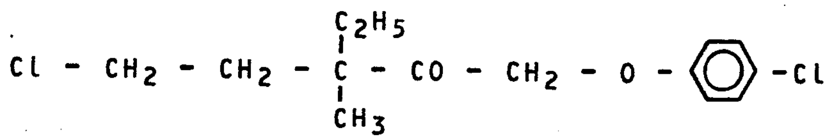 (V-2)

79,7 g (0,62 Mol) 4-Chlorphenol und 85,6 g (0,62 Mol) Kaliumcarbonat in 600 ml Toluol werden 2 Stunden unter Rückfluß am Wasserabscheider erhitzt. Bei 70°C versetzt man anschließend mit 104 g (0,53 Mol) 1,5-Dichlor-3-ethyl-3-methyl-2-pentanon in 100 ml Toluol. Das Reaktionsgemisch wird 5 Stunden bei 100°C gerührt, danach läßt man abkühlen und verrührt mit 500 ml Wasser. Die organische Phase wird abgetrennt, mit verdünnter Natronlauge und mit Wasser gewaschen, über Natriumsulfat getrocknet und eingeengt. Man erhält 110 g (71,8 % der Theorie) 1-(4-chlorphenoxy)-5-chlor-3-ethyl-3-methyl-2-pentanon vom Brechungsindex $n_D^{20}$ = 1,5092.

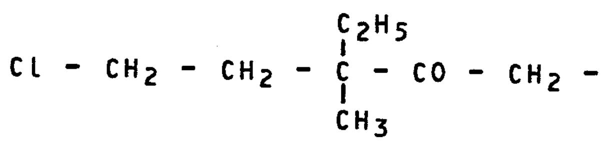 (III-2)

64 g (0,4 Mol) 2-Chlormethylen-3-ethyl-3-methyl-tetrahydrofuran werden bei 10 bis 20°C unter Eiskühlung mit Chlorwasserstoffgas gesättigt. Nach 2-stündigem Stehen bei Raumtemperatur wird überschüssiger Chlorwasserstoff abgezogen und der Rückstand wird destilliert. Man erhält 60 g (81 % der Theorie) 1,5-Dichlor-3-ethyl-3-methyl-2-pentanon vom Siedepunkt 92-101°C/0,05 mbar.

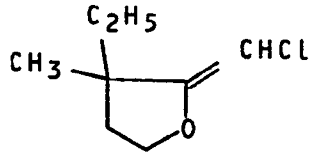

(VI-2)

1260 g (5 Mol) 3-Ethyl-3-methyl-1,1,1,5-tetrachlorpentan und 902 g (11 Mol) wasserfreies Natriumacetat werden in 4 l Dimethylformamid 10 Stunden unter Rückfluß erhitzt. Danach werden 2,2 l (11 Mol) 30 %ige Natriummethylat-Lösung zugetropft und 4 Stunden unter Rückfluß erhitzt. Die Reaktionslösung wird mit Wasser verdünnt und mit Methylenchlorid ausgeschüttelt. Die organische Phase wird über Natriumsulfat getrocknet und eingeengt. Der Rückstand wird destilliert. Man erhält 640 g (80 % der Theorie) 2-Chlormethylen-3-ethyl-3-methyl-tetrahydrofuran vom Siedepunkt 100°C/16 mbar.

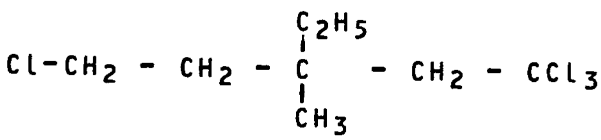

(IX-1)

2180 g (22,5 Mol) 1,1-Dichlorethen werden bei -70°C vorgelegt, 75 g gepulvertes Aluminiumchlorid werden zugesetzt und danach 1160 g (7,5 Mol) 1,3-Dichlor-3-methylpentan eingetropft. Man rührt das Reaktionsgemisch 4 Stunden bei -70°C und gießt dann auf Eis und verdünnte Salzsäure. Man extrahiert mit Methylenchlorid, trocknet die organische Phase über Natriumsulfat und engt ein.

Der Rückstand wird destilliert. Man erhält 1572 g (83 % der Theorie) 3-Ethyl-3-methyl-1,1,1,5-tetrachlorpentan vom Siedepunkt 85°C/0,1 mbar.

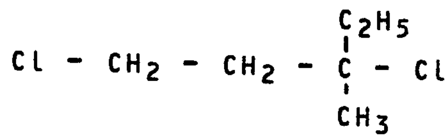

245 g (24,26 Mol) 3-Methyl-1-penten-3-ol werden unter kräftigem Rühren zu 4 l konzentrierter Salzsäure gegeben. Danach wird bei 20°C Chlorwasserstoffgas bis zur Sättigung eingeleitet. Die organische Phase wird abgetrennt, über Natriumsulfat getrocknet und fraktioniert. Man erhält 2323 g (62 % der Theorie) 1,3-Dichlor-3-methylpentan vom Siedepunkt 58-64°C/16 mbar.

Beispiel 4

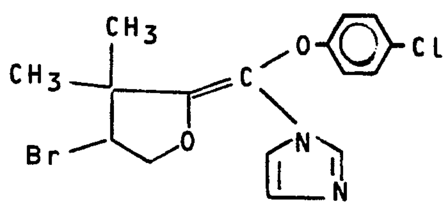

35 g (0,5 Mol) Imidazol und 103,5 g (0,75 Mol) Kaliumcarbonat werden bei 60°C in 700 ml Toluol gelöst. Zu dieser Lösung werden innerhalb von 20 Minuten 132 g (0,269 Mol) 1-(4-Chlorphenoxy)-3,3-dimethyl-1,4,5-tribrom-2-pentanon in 100 ml Toluol getropft. Man läßt das Reaktionsgemisch 10 Stunden bei 90°C nachrühren,

kühlt ab und versetzt mit Wasser. Die organische Phase wird abgetrennt, mit Wasser gewaschen, über Natriumsulfat getrocknet und eingeengt. Der Rückstand wird säulenchromatographisch gereinigt (Kieselgel; Essigester/Cyclohexan = 3:1). Man erhält 10,6 g (10 % der Theorie) (4-Chlorphenoxy)-(imidazol-1-yl)-(4-brom-3,3-dimethyltetrahydrofuran-2-yliden)-methan vom Brechungsindex $n_D^{20}$ = 1,5837.

Herstellung des Ausgangsproduktes

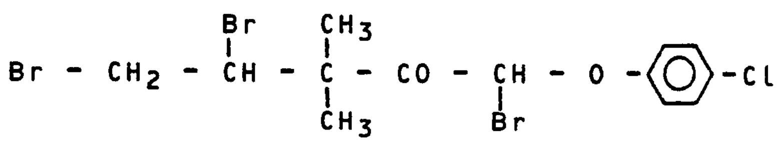

(II-3)

107 g (0,269 Mol) 1-(4-Chlorphenoxy)-4,5-dibrom-3,3-dimethyl-2-pentanon werden in 400 ml Chloroform gelöst. Man läßt bei Raumtemperatur 43 g (0,269 Mol) Brom so zutropfen, daß sich die Lösung immer entfärbt. Anschließend wird 30 Minuten bei Raumtemperatur nachgerührt, mit Wasser gewaschen, über Natriumsulfat getrocknet und eingeengt. Man erhält 128 g (99 % der Theorie) 1-(4-Chlorphenoxy)-3,3-dimethyl-1,4,5-tribrom-2-pentanon vom Brechungsindex $n_D^{20}$ = 1,5472.

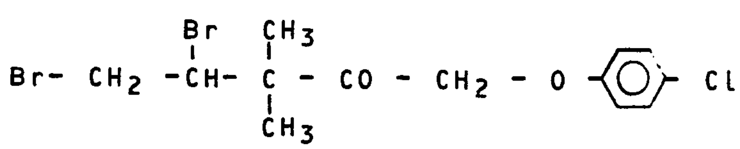

(V-3)

70,7 g (0,55 Mol) 4-Chlorphenol und 75,9 g (0,55 Mol) Kaliumcarbonat werden in 500 ml Toluol 2 Stunden unter Rückfluß am Wasserabscheider erhitzt. Bei 70°C versetzt man anschließend mit 133 g (0,433 Mol) 1-Chlor-4,5-dibrom-3,3-dimethyl-2-pentanon in 100 ml Toluol. Das Reaktionsgemisch wird 5 Stunden bei 100°C gerührt, danach läßt man abkühlen, wäscht mit Wasser und verdünnter Natronlauge, trocknet über Natriumsulfat und engt ein. Man erhält 109,2 g (63,3 % der Theorie) 1-(4-Chlorphenoxy)-4,5-dibrom-3,3-dimethyl-2-pentanon vom Brechungsindex $n_D^{20}$ = 1,5363.

$$Br-CH_2-\overset{Br}{\overset{|}{CH}}-\underset{\underset{CH_3}{|}}{\overset{\overset{CH_3}{|}}{C}}-CO-CH_2Cl \qquad (III-3)$$

59 g (0,4 Mol) 1-Chlor-3,3-dimethyl-4-penten-2-on werden in 200 ml Methylenchlorid vorgelegt. Bei -70°C wird eine Lösung von 320 g (0,4 Mol) Brom in 200 ml Methylenchlorid zugetropft. Man läßt 15 Minuten bei Raumtemperatur nachrühren und engt durch Abdestillieren des Lösungsmittels im Vakuum ein. Man erhält 121 g (98,7 % der Theorie) rohes 1-Chlor-4,5-dibrom-3,3-dimethyl-2-pentanon, das direkt weiter umgesetzt wird.

In entsprechender Weise und gemäß den angegebenen Verfahrensbedingungen können die folgenden Verbindungen der allgemeinen Formel (I)

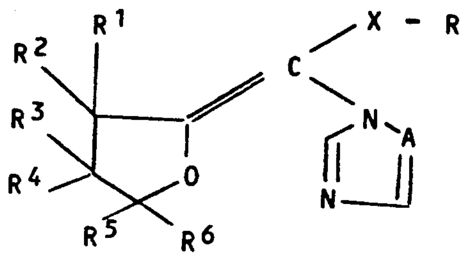

(I)

erhalten werden:

| Bsp. Nr. | $R^1$ | $R^2$ | $R^3$ | $R^4$ | $R^5$ | $R^6$ | X | R | A | Fp(°C) bzw. $n_D^{20}$ |
|---|---|---|---|---|---|---|---|---|---|---|
| 5 | $C_2H_5$ | $CH_3$ | H | H | H | H | O | 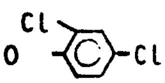 | CH | 1,5524 |
| 6 | $C_2H_5$ | $CH_3$ | H | H | H | H | O | 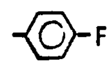 | CH | 1,5393 |
| 7 | $C_2H_5$ | $CH_3$ | H | H | H | H | O | 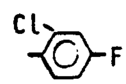 | CH | 78 |
| 8 | $CH_3$ | $CH_3$ | H | H | H | H | S | 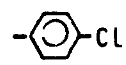 | N | 104 (Form A) |
| 9 | $CH_3$ | $CH_3$ | H | H | H | H | S | 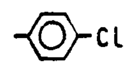 | N | 176 (Form B) |
| 10 | $CH_3$ | $CH_3$ | H | H | H | H | $SO_2$ | 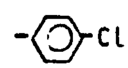 | N | 134 |
| 11 | $C_2H_5$ | $CH_3$ | H | H | H | H | S | 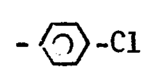 | CH | 200 (XHCl) |
| 12 | $C_2H_5$ | $CH_3$ | H | H | H | H | O | 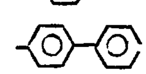 | CH | 1,5906 |

Form A und B: die beiden möglichen geometrischen Isomeren.

Verwendungsbeispiele

In den nachfolgenden Beispielen werden die nachstehend angegebenen Verbindungen als Vergleichssubstanzen eingesetzt:

(A) 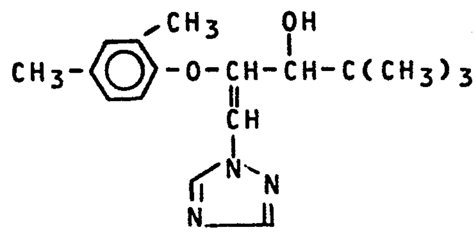

(B) 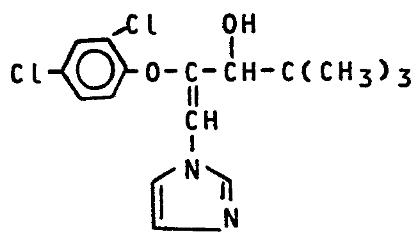

(C) 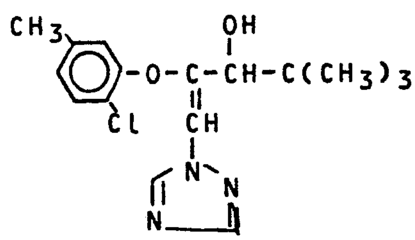

(D) 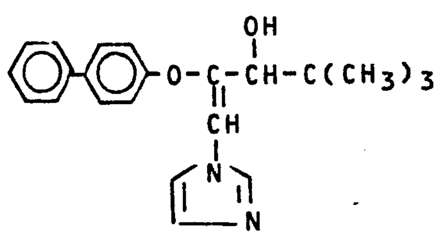

(E) 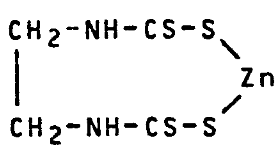

Beispiel A

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 3 und 5.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 5, 3, 4 und 7.

Beispiel C

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 3, 4 und 7.

Beispiel D

Pellicularia-Test (Reis)

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 5, 3, 4 und 7.

Beispiel E

Erysiphe-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 3, 5 und 4.

Beispiel F

Botrytis-Test (Bohnen) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß folgender Herstellungsbeispiele: 4 und 7.

Patentansprüche

1. Substituierte Azolyl-tetrahydrofuran-2-yliden-methan-Derivate der allgemeinen Formel (I)

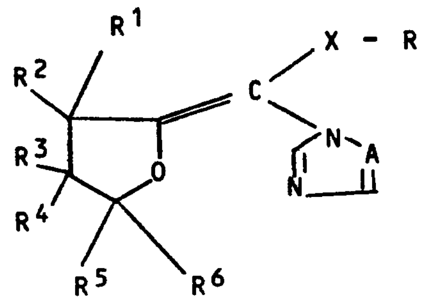

(I)

in welcher

A für ein Stickstoffatom oder die CH-Gruppe steht;

X für Sauerstoff, Schwefel, die SO- oder $SO_2$-Gruppe steht;

R für gegebenenfalls substituiertes Aryl steht;

$R^1$ bis $R^6$ gleich oder verschieden sind und für Wasserstoff, Alkyl, Halogenalkyl oder Halogen stehen, wobei maximal bis zu 3 Substituenten für Halogen bzw. Halogenalkyl stehen, mit der Maßgabe, daß $R^1$ und $R^2$ nicht gleichzeitig für Methyl stehen, wenn X für Sauerstoff steht;

$R^1$ und $R^2$ gemeinsam mit dem Kohlenstoffatom, an das sie gebunden sind, für gegebenenfalls substituiertes Cycloalkyl stehen und

0141995

$R^2$ und $R^3$ gemeinsam mit den Kohlenstoffatomen, an die sie gebunden sind, für gegebenenfalls substituiertes Cycloalkyl stehen,

und deren Säureadditions-Salze und Metallsalz-Komplexe.

2. Substituierte Azolyl-tetrahydrofuran-2-yliden-methan-Derivate gemäß Anspruch 1, wobei in der Formel (I)

A für ein Stickstoffatom oder die CH-Gruppe steht;

X für Sauerstoff, Schwefel, die SO- oder $SO_2$-Gruppe steht;

R für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Halogenatomen, Alkoxycarbonyl mit 1 bis 4 Kohlenstoffatomen im Alkoxyteil, Alkoximinoalkyl mit 1 bis 4 Kohlenstoffatomen im Alkoxyteil und 1 oder 2 Kohlenstoffatomen im Alkylteil, Nitro, Cyano oder gegebenenfalls durch Halogen und/oder Alkyl mit 1 oder 2 Kohlenstoffatomen substituiertes Phenyl oder Phenoxy;

$R^1$ bis $R^6$ die gleich oder verschieden sein können, für Wasserstoff, geradkettiges oder verzweigtes Alkyl mit 1 bis 4 Kohlenstoffatomen oder für Halogenalkyl mit 1 oder 2 Kohlenstoffatomen und 1 bis 5 gleichen oder verschiedenen Halogenatomen stehen, wobei maximal bis zu 3 Substituenten für Halogenalkyl stehen, mit der Maßgabe, daß $R^1$ und $R^2$ nicht gleichzeitig für Methyl stehen, wenn X für Sauerstoff steht;

$R^3$ bis $R^6$, die gleich oder verschieden sein können, außerdem für Halogen stehen, wobei maximal bis zu 3 Substituenten diese Bedeutung haben;

$R^1$ und $R^2$ gemeinsam mit dem Kohlenstoffatom, an das sie gebunden sind, für gegebenenfalls durch Alkyl mit 1 oder 2 Kohlenstoffatomen substituiertes Cycloalkyl mit 4 bis 7 Kohlenstoffatomen stehen, und

$R^2$ und $R^3$ gemeinsam mit den Kohlenstoffatomen, an die sie gebunden sind, für gegebenenfalls durch Alkyl mit 1 oder 2 Kohlenstoffatomen substituiertes Cycloalkyl mit 4 bis 7 Kohlenstoffatomen stehen,

und deren Säureadditions-Salze und Metallsalzkomplexe.

3. Substituierte Azolyl-tetrahydrofuran-2-yliden-methan-Derivate gemäß Anspruch 1, wobei in der Formel (I)

A für ein Stickstoffatom oder die CH-Gruppe steht;

X für Sauerstoff, Schwefel, die SO- oder $SO_2$-Gruppe steht;

R für gegebenenfalls einfach bis dreifach, gleich oder verschieden substituiertes Phenyl steht, wobei als Substituenten genannt seien: Halogen, Alkyl mit 1 bis 4 Kohlenstoffatomen, Alkoxy und Alkylthio mit jeweils 1 oder 2 Kohlenstoffatomen, Halogenalkyl, Halogenalkoxy und Halogenalkylthio mit jeweils 1 oder 2 Kohlenstoffatomen und 1 bis 5 gleichen oder verschiedenen Fluor- und Chloratomen, Alkoxycarbonyl mit 1 bis 4 Kohlenstoffatomen im Alkylteil, Alkoximinoalkyl mit 1 bis 4 Kohlenstoffatomen im Alkoxyteil und 1 oder 2 Kohlenstoffatomen im Alkylteil, Nitro, Cyano oder gegebenenfalls durch Halogen und/oder Alkyl mit 1 oder 2 Kohlenstoffatomen substituiertes Phenyl oder Phenoxy;

$R^1$ bis $R^6$, die gleich oder verschieden sein können, für Wasserstoff, geradkettiges oder verzweigtes

Alkyl mit 1 bis 4 Kohlenstoffatomen oder für Halogenalkyl mit 1 oder 2 Kohlenstoffatomen und 1 bis 5 gleichen oder verschiedenen Fluor- und Chloratomen stehen, wobei maximal bis zu 3 Substituenten für Halogenalkyl stehen, mit der Maßgabe, daß $R^1$ und $R^2$ nicht gleichzeitig für Methyl stehen, wenn X für Sauerstoff steht;

$R^3$ bis $R^6$, die gleich oder verschieden sein können, außerdem für Halogen stehen, wobei maximal bis zu 3 Substituenten diese Bedeutung haben;

$R^1$ und $R^2$ gemeinsam mit dem Kohlenstoffatom, an das sie gebunden sind, für gegebenenfalls durch Alkyl mit 1 oder 2 Kohlenstoffatomen substituiertes Cycloalkyl mit 4 bis 7 Kohlenstoffatomen stehen, und

$R^2$ und $R^3$ gemeinsam mit den Kohlenstoffatomen, an die sie gebunden sind, für gegebenenfalls durch Alkyl mit 1 oder 2 Kohlenstoffatomen substituiertes Cycloalkyl mit 4 bis 7 Kohlenstoffatomen stehen,

und deren Säureadditions-Salze und Metallsalz-Komplexe.

4. Substituierte Azolyl-tetrahydrofuran-2-yliden-methan-Derivate gemäß Anspruch 1, wobei in der Formel (I)

 

A für ein Stickstoffatom oder die CH-Gruppe steht,

X für Sauerstoff, Schwefel, die SO- oder $SO_2$-Gruppe steht;

R für gegebenenfalls einfach bis dreifach, gleich oder verschieden substituiertes Phenyl steht, wobei als Substituenten genannt seien: Fluor, Chlor, Brom, Methyl, Ethyl, tert.-Butyl, Methoxy, Methylthio, Trifluormethyl, Trifluormethoxy, Trifluormethylthio, Methoxycarbonyl, Ethoxycarbonyl, Methoximinomethyl, 1-Methoximinoethyl, Nitro, Cyano, gegebenenfalls durch Chlor und/oder Methyl substituiertes Phenyl oder Phenoxy;

$R^1$ bis $R^6$, die gleich oder verschieden sein können, für Wasserstoff, Methyl, Ethyl oder Halogenmethyl mit 1 bis 3 gleichen oder verschiedenen Fluor- und Chloratomen, stehen, wobei maximal drei Substituenten für Halogenmethyl stehen, mit der Maßgabe, daß $R^1$ und $R^2$ nicht gleichzeitig für Methyl stehen, wenn X für Sauerstoff steht;

$R^3$ bis $R^6$, die gleich oder verschieden sein können, außerdem für Fluor, Chlor oder Brom stehen, wobei maximal bis zu drei Substituenten diese Bedeutung haben;

$R^1$ und $R^2$ gemeinsam mit dem Kohlenstoffatom, an das sie gebunden sind, für Cyclopentyl oder Cyclohexyl stehen; und

$R^2$ und $R^3$ gemeinsam mit den Kohlenstoffatomen, an die sie gebunden sind, für Cyclopentyl oder Cyclohexyl stehen,

und deren Säureadditions-Salze und Metallsalz-Komplexe.

5. Verfahren zur Herstellung von substituierten Azolyl-tetrahydrofuran-2-yliden-methan-Derivaten der Formel (I)

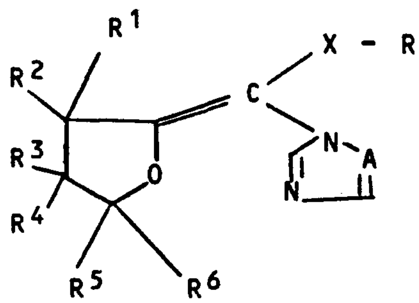

(I)

in welcher

A für ein Stickstoffatom oder die CH-Gruppe steht;

X für Sauerstoff, Schwefel, die SO- oder $SO_2$-Gruppe steht;

R für gegebenenfalls substituiertes Aryl steht;

$R^1$ bis $R^6$ gleich oder verschieden sind und für Wasserstoff, Alkyl, Halogenalkyl oder Halogen stehen, wobei maximal bis zu 3 Substituenten für Halogen bzw. Halogenalkyl stehen, mit der Maßgabe, daß $R^1$ und $R^2$ nicht gleichzeitig für Methyl stehen, wenn X für Sauerstoff steht;

$R^1$ und $R^2$ gemeinsam mit dem Kohlenstoffatom, an das sie gebunden sind, für gegebenenfalls substituiertes Cycloalkyl stehen und

$R^2$ und $R^3$ gemeinsam mit den Kohlenstoffatomen, an die sie gebunden sind, für gegebenenfalls substituiertes Cycloalkyl stehen,

und deren Säureadditions-Salze und Metallsalz-Komplexe, dadurch gekennzeichnet, daß man Halogen-(thio)ether-ketone der Formel (II)

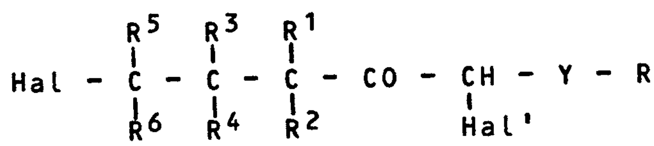

(II)

in welcher

R und $R^1$ bis $R^6$ die oben angegebene Bedeutung haben,

Y für Sauerstoff oder Schwefel steht und

Hal und Hal' für Halogen stehen,

mit Imidazol oder 1,2,4-Triazol in Gegenwart eines Verdünnungsmittels und in Gegenwart eines Säurebindemittels umsetzt; und gegebenenfalls die so erhaltenen substituierten Azolyl-tetrahydrofuran-2-yliden-methan-Derivate der Formel (Ia)

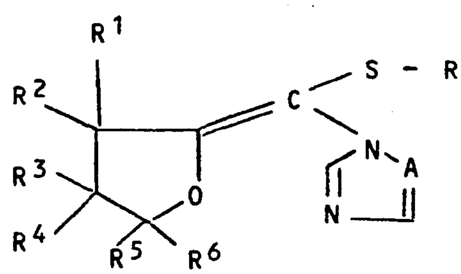

(Ia)

in welcher

A,R und $R^1$ bis $R^6$ die oben angegebene Bedeutung haben,

nach bekannten Methoden in üblicher Weise oxidiert und gegebenenfalls an die so erhaltenen Verbindungen der Formel (I) anschließend eine Säure oder ein Metallsalz addiert.

6. Fungizide Mittel, gekennzeichnet durch einen Gehalt an mindestens einem substituierten Azolyl-tetrahydrofuran-2-yliden-methan-Derivat der Formel (I) gemäß Ansprüchen 1 und 5.

7. Verfahren zur Bekämpfung von Pilzen, dadurch gekennzeichnet, daß man substituierte Azolyl-tetrahydrofuran-2-yliden-methan-Derivate der Formel (I) gemäß Ansprüchen 1 und 5 auf Pilze oder ihren Lebensraum einwirken läßt.

8. Verwendung von substituierten Azolyl-tetrahydrofuran-2-yliden-methan-Derivaten der Formel (I) gemäß Ansprüchen 1 und 5 als Pflanzenschutzmittel.

9. Verwendung von substituierten Azolyl-tetrahydrofuran-2-yliden-methan-Derivaten der Formel (I) gemäß Ansprüchen 1 und 5 zur Bekämpfung von Pilzen.

10. Verfahren zur Herstellung von fungiziden Mitteln, dadurch gekennzeichnet, daß man Azolyl-tetrahydrofuran-2-yliden-methan-Derivate der Formel (I) gemäß Ansprüchen 1 und 5 mit Streckmitteln und/ oder oberflächenaktiven Mitteln vermischt.